(19) Europäisches Patentamt
European Patent Office
Office européen des brevets

(11) EP 0 564 676 B1

(12) **EUROPEAN PATENT SPECIFICATION**

(45) Date of publication and mention of the grant of the patent:
**30.12.1998 Bulletin 1998/53**

(51) Int Cl.[6]: **C07D 215/48**, C07D 215/50, G02F 1/17, G02B 5/30

(21) Application number: **92106013.3**

(22) Date of filing: **07.04.1992**

(54) **Light polarizing material based on quinaldic acid derivatives**

Licht-polarisierendes Material basierend auf Chinaldinsäurederivaten

Matériaux de polarisation de la lumière basés sur des dérivés de l'acide quinaldique

(84) Designated Contracting States:
**AT BE CH DE DK ES FR GB GR IT LI LU MC NL PT SE**

(43) Date of publication of application:
**13.10.1993 Bulletin 1993/41**

(73) Proprietor: **RESEARCH FRONTIERS INCORPORATED**
**Woodbury New York 11797 (US)**

(72) Inventors:
- **Check III, Joseph Aloysius**
  **Massapequa, New York 11758 (US)**
- **Saxe, Robert L.**
  **New York, New York 10021 (US)**

(74) Representative: **Dr. Elisabeth Jung**
**Dr. Jürgen Schirdewahn Dipl.-Ing. Claus Gernhardt**
**Postfach 40 14 68**
**80714 München (DE)**

(56) References cited:
**EP-A- 403 711**
**EP-A- 425 344**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 564 676 B1

## Description

This invention relates to light-polarizing materials, to set suspensions and fluid suspensions thereof, and to light valves containing such fluid suspensions.

Light-polarizing materials, such as colloidal suspensions of herapathite and herapathite-like light-polarizing crystals, are described in U.S. Patents 1,951,664 (Land) and 2,178,996 (Land), respectively. U.S. Patent 2,237,567 (Land) discloses the production of light-polarizing material in sheet form by various methods including application of a solution of iodine and an iodide to a sheet of polyvinyl alcohol which had been previously stretched to orient the molecules therein. Numerous other patents relating to light-polarizing materials, set suspensions thereof and laminated products derived therefrom and uses thereof are in the art including, for example, U.S. Patent Nos. 2,041,138 (Land), 2,078,254 (Land), 2,168,220 (Land), 2,168,221 (Land), 2,185,018 (Sauer), 2,230,262 (Pollack), 2,246,087 (Bailey et al), 2,256,108 (Blake), 2,263,249 (Rogers), 2,306,108 (Land et al), 2,328,219 (Land), and 2,375,963 (Thomas). U.K. Patent 433,455 discloses the use of particles of purpureocobalt-chloridesulphateperiodide in the formation of light-polarizing bodies. These and the other patents and prior art referred to in this specification are incorporated herein by reference thereto.

At present, important uses for laminated set suspensions of light-polarizing materials, often referred to as "sheet polarizers", include lenses for polarized sunglasses, components of the twisted nematic and other types of liquid crystal displays and filters of various types including contrast enhancement filters for use in conjunction with light emissive displays. However, the sheet polarizers thus employed are well known to be frequently subject to degradation due to high levels of heat, ultraviolet radiation and/or especially moisture.

Fluid suspensions of light-polarizing and other materials have been used in light valves, comprising a cell containing a fluid suspension of minute particles which can be oriented by an electric or magnetic field to change the transmission of light through the suspension. See for example, U.S. Patent Nos. 3,708,219 (Forlini et al), 3,743,382 (Rosenberg), 4,078,856 (Thompson et al), 4,113,362 (Saxe et al), 4,164,365 (Saxe), 4,407,565 (Saxe), and 4,422,963 (Thompson et al).

U.S. Patent 4,131,334 (Witte et al) describes a process for forming light-polarizing particles by hydrogenation of a nitrogen-containing organic compound, which is then reacted with an appropriate acid to form a salt. The salt may then be reacted, usually with iodine and an inorganic iodide, to produce stable polyiodide particles.

An object of the present invention is to provide light-polarizing materials that have high stability with respect to ultraviolet radiation, elevated temperatures and high levels of moisture.

Polyhalides, including polyiodides, have been known for quite some time. A polyiodide is a complex of iodine atoms and an inorganic or organic matrix. Godina et al discuss polyiodides and other polyhalides in detail in J. Gen. Chem. USSR, 20, (1950), pages 1005-1016. Among the known polyiodides is the light-polarizing crystalline material, herapathite, which is formed by reaction of quinine bisulfate, iodine and HI. Salts of other members of the quinine alkaloid family also form light-polarizing polyiodides by reaction with iodine and HI, such as cinchonidine bisulfate. In these materials, the elemental iodine combines with the alkaloid acid salt in the form of the polyiodide anion, which has been variously described as $I_3^-$ by Godina et al and as $I_5^-$ by Teitelbaum et al, JACS, 100 (1978) pages 3215-3217. Godina et al show that the polyiodide anion is formed by reaction between iodine and HI, e.g.

$$(1) \qquad I_2 + HI = H^+ + I_3^-$$

Likewise, the $I_5^-$ polyiodide anion would be formed by the reaction

$$(2) \qquad 2I_2 + HI = H^+ + I_5^-$$

Godina et al explain that light-polarizing polyiodides comprise the polyiodide anion and the acid salt of quinine and the like as the cation. However, polyiodides can also be formed without any apparent cation being present, such as the starch-iodine complex and the stretched or oriented polyvinyl alcohol-iodine complex. Teitelbaum et al report that the starch-iodine complex contains adsorbed iodine in the form of chains of iodine within the amylase component of starch, the chains being made up of $I_5^-$ polyiodide anions as the dominant species. Godina et al theorize that herapathite, starch-iodine and oriented PVA-iodine complex are "adsorbing polyiodides" in which molecular iodine is adsorbed in layers on the polyiodide chains.

In the references EP-A 0 403 711 and EP-A 0 425 344 are disclosed light polarizing materials and suspensions thereof for different uses in liquid suspensions, set suspensions and light valves.

The light-polarizing material of the present invention is a complex obtained by reacting (i) elemental iodine, (ii) a hydrohalide acid and/or an ammonium or alkali metal or alkaline earth metal halide and (iii) a compound of formula I below. This complex contains adsorbed molecular iodine. We believe that the complex also contains the polyiodide anion, $I_x^-$, where x is 3 or 5, since Godina et al and Teitelbaum et al both report that the polyiodide anion is formed by reaction between (i) elemental iodine and (ii) an iodide. Moreover, Godina et al report that crystals containing adsorbed molecular iodine and the polyiodide anion are light-polarizing.

In the Examples that follow, light polarizing materials are prepared by reacting a compound I with iodine

and an iodide, bromide or chloride. In such cases, the respectively anions would be

-I-I-$\overline{I}$-I-I-

-I-I-$\overline{B}$r-I-I

-I-I-$\overline{C}$I-I-I using the structure elucidated by Teitelbaum et al as a model.

Godina et al report that light-polarizing complexes containing adsorbed molecular iodine cannot be defined stoichiometrically by structural formula. Hence, the light-polarizing material of the present invention is defined in product-by-process format.

Compounds I that are useful in forming the light-polarizing materials of the invention have the formula:

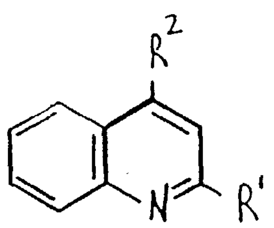

wherein $R^1$ is carboxy and $R^2$ is methoxy, hydroxy or lower alkyl substituted by hydroxy or methoxy. The term "lower alkyl" includes straight or branched chain alkyl, such as methyl, ethyl, propyl, isopropyl, butyl, isobutyl, t-butyl and the like. In the present invention, lower alkyl has from 1 to 6 carbon atoms, preferably 1 to 4 carbon atoms.

Compound I is known per se or may be isomers, homologues or analogs of known compounds and may be prepared analogously to such known compounds.

Useful compounds of formula I include:

Compound

1 - 4-methoxy quinaldic acid.
2 - 4-hydroxy quinaldic acid.

The light-polarizing materials of this invention are formed by reacting a compound of formula I with elemental iodine and a hydrohalide acid and/or an ammonium, alkali metal or alkaline earth metal halide, in a suitable solvent, such as an alcohol or etheralcohol. See U. S. Patent Nos. 1,951,661, 2,176,516 and 2,289,712. The halide is usually an iodide, but can also be a bromide or chloride. Preferably, the reaction to form the polyhalide takes place in the presence of a protective colloid, such as nitrocellulose or a copolymer as disclosed in U.S. Patent No. 4,164,365, issued August 14, 1979. It is presently preferred to provide compound I in a first solution and a mixture of iodine and ammonium or alkali metal or alkaline earth metal halide in a second solution, but, if desired, the halide can be in either or both of the solutions. The solutions are then mixed together, and the polyhalides are readily formed even at room temperature. Light-polarizing polyhalide crystals are then recovered by any suitable technique, such as by filtering and the like.

For use in a light valve, the polyhalide particles are suspended in a liquid suspending medium. As is known, the liquid suspending medium may be virtually any electrically resistive liquid so long as it suspends the particles and dissolves the polymeric stabilizer. Preferably, the liquid suspending medium has a relatively high electrical resistivity and low vapor pressure, and does not degrade or attack the particles or other components of the suspension. See e.g. U.S. Patents 4,270,841 and 4,407,565 to Saxe.

For use in set suspensions, the polyhalide particles are dispersed or distributed throughout a sheet formed of suitable film-forming material, such as cellulose acetate or polyvinylalcohol or the like. See e.g. U.S. Patents 2,178,996 and 2,041,138.

**Examples 1 and 2**

Approximately 1.5 g. of compound 1 was dissolved in 13 g. of a solution comprising 10 g. of 2-ethoxyethanol and 3 g. of methanol, and that solution was then mixed with a solution of 10 g. of n-propanol in which 0.22 g. of calcium iodide and 1.3 g. of iodine were dissolved. Blue-colored light-polarizing crystals formed readily. The same procedure was followed and similar results observed for compound 2.

**Examples 3 and 4**

Examples 1 and 2 are repeated but with an effective amount of $CaBr_2$ substituted for $CaI_2$. Light-polarizing crystals having a somewhat different bluish color are formed.

**Examples 5 and 6**

Examples 1 and 2 are repeated but with an effective amount of $CaCl_2$ substituted for $CaI_2$. Light-polarizing crystals having a somewhat different bluish color are formed.

**Examples 7 and 8**

Approximately 1.5 g. of compound 1 was dissolved in 13 g. of a solution comprising 10 g. of 2-ethoxyethanol and 3 g. of methanol and that solution was then mixed with a solution formed by dissolving 0.58 g. of potassium iodide (KI) in about 0.58 g. of water to which a solution of 1.3 g. of iodine in 10 g. of n-propanol was added. Blue-colored light-polarizing crystals formed readily. The same procedure was followed and similar results observed for compound 2. These examples illustrate that the particles of the present invention will tolerate water and moisture since they were in fact made and remained undissolved in the presence of water.

**Examples 9 and 10**

Examples 7 and 8 are repeated but using 0.62 g. of CsI in place of 0.58 g. of KI, with similar results.

Although individual halides are employed in the above examples, mixtures of iodides and other halides, may be advantageously used in such reactions as those described above. For example, equimolar amounts of CsI and $CaI_2$ in solution can be reacted with Compound I and $I_2$ in solution to form fine polarizing crystals.

**Example 11**

Approximately 1 g of compound 1 was combined with 0.31 g. of $CaI_2$ and 0.25 g. of $I_2$ in a mixed solvent comprised of 20 g of ethyl benzoate and 130 g. of ethyl acetate. The reaction mixture was subjected to 1 hour of ultrasonic agitation during which small blue needle-shaped polarizing crystals formed.

Some of the compounds I used in the present invention are known to form metal salts and/or to be metal-chelating compounds. Accordingly, one possible explanation for the formation of the light-polarizing materials of this invention is that when the compounds I are reacted with iodine and a halide, the halide and iodine enter into the reaction in an ionic form. For example, if the halide is calcium iodide, $CaI_2$, iodine may enter the reaction as $Ca^{+2}(I_x)_2^-$, with the positively charged calcium ion being chelated by compounds I and the $(I_x)^-$ anion being bonded to the positive calcium ion, thereby forming a polyiodide crystal. While this explanation seems reasonable, it is not intended that this application be bound by this theory.

**Example 12**

Preparation of suspension of 4-methoxy quinaldic acid polyiodide nitrocellulose complex.

To a mixed solvent comprised of 130 g. of ethyl acetate and 20 g. of ethyl benzoate was added 1 g. of 4-methoxy quinaldic acid together with 0.31 g. of calcium iodide, 0.25 g. of iodine and 5 g. of dry low viscosity (18-25 cps) nitrocellulose. The reaction mixture was shaken for 15 minutes and then ultrasonically agitated for 10 hours. Small blue needle-shaped polarizing crystals formed over the course of the ultrasonic agitation. The resulting suspension was centrifuged for 1 hour at 2500 rpm and the sediment discarded. The supernatant liquid was further centrifuged for 1 hour at 14,500 rpm, the supernatant was discarded and the sediment was then resuspended in ethyl acetate and an effective amount of a suitable polymer solution added thereto, e. g. 10-15 g. of a 15% solution of a 96.75%/3.25% copolymer of neopentyl acrylate/methylol acrylamide dissolved in neopentyl neopentanoate may be added. This new suspension is placed in a vacuum apparatus for approximately five hours to evaporate off nearly all of the ethyl acetate. Part of any other solvent present that does not have too high a boiling point may also evaporate. The suspension is then diluted to the extent desired with Halocarbon Oil type 0.8/100 (manufactured by Halocarbon Products, Hackensack, New Jersey) and any other desired solvents, such as neopentyl neopentanoate, to provide the desired off-state optical density and response time. Additional polymer may be added.

Such suspensions have been subjected to intense ultraviolet radiation with excellent results. After about one month at about one foot distance from an ultraviolet lamp, a test cell containing a suspension of 4-methoxy quinaldic acid polyiodide showed no substantial change in properties.

Liquid suspensions of the type described above can be used in light valves which utilize an AC electric field to orient the particles in said suspensions to change and/or control the transmission of light through the suspension. Such light valves can be used, for example, as variable transmission windows, filters, mirrors, and eyeglasses, and as electronic alphanumeric and graphic image displays.

By modifying the composition of the suspension, however, it is possible to produce what is known in the prior art as a set suspension, rather than a fluid suspension or liquid suspension usable in a light valve as described above. A set suspension of the particles of the present invention would comprise, for example, a light-polarizing sheet or film in which said particles would be incorporated along with other materials.

There are many processes known in the art for producing light-polarizing sheets and films. For example, U.S. Patent 2,178,996 discloses a process for forming certain light-polarizing particles, mixing said particles into a dispersion medium which may include cellulose acetate, and subjecting the dispersion of particles to flow or extrusion or stretch, or rolling, so that the needle axis of the dispersed polarizing crystals may be oriented to substantial parallelism and a thin, sheet-like polarizing body produced. U.S. Patent 2,041,138 discloses that polarizing bodies may preferably be made in the form of a relatively thin sheet or film comprising the suspending medium and the minute particles dispersed therein. If desired, the polarizing body may itself be permanently or detachably fixed to a suitable support, preferably transparent, as for example, to a plate of glass or to a sheet of celluloid. Such a support may be desirable with conditions where it is found that the polarizing body itself may require some form of protection. It also discloses the use of asymmetric particles, the flowing of the medium that includes said particles past an edge, and retaining said particles in an oriented position by setting or hardening said medium.

U. S. Patent 2,168,220 discloses information relating to polarizing material sold under the trade name "Polaroid". Use of plasticizers, adhesives and various types of laminations and methods for forming said laminations are disclosed.

Numerous types of polarizing films and uses for polarizers are disclosed in U.S. Patent 2,246,087 including, for example, use in windshields, windows, eyeglasses, goggles, sunglasses, camera lenses, microscopes, mirrors and in connection with three dimensional movies.

A process for transferring light-polarizing films from one support to another and various materials used in connection therewith are disclosed in U.S. Patent 2,256,108.

The information available from any of the aforesaid patents and from numerous other patents and other sources known in the art can be used to make light-polarizing set suspensions, films and sheets which include particles oriented in substantial parallelism, and light-polarizing bodies and products made therefrom.

However, many light polarizers in commercial use today do not incorporate films or sheets having solid discrete particles oriented in parallel therein, but rather use a sheet of polyvinyl alcohol polyiodide which has its optic axis in the plane of the sheet and which transmits with substantially no absorption only light vibrating substantially perpendicularly to its optic axis, as described in U. S. Patent 2,237,567 and 2,375,963 and other sources known in the art. The commercially available polarizers are known to be susceptible to degradation when subjected for prolonged periods to harsh environmental conditions such as high temperatures, high humidity, ultraviolet radiation and especially combinations of such conditions.

Despite the problems of commercially available sheet polarizers with respect to environmental degradation, it may be preferable or desirable from a manufacturing viewpoint to react a stretched sheet of polymer with dyes or stains or with iodine and an iodide to form a light-polarizing complex, rather than to use a plurality of individual polarizing crystals as previously described. To this end, useful embodiments of the present invention also include compounds comprising compounds I of this invention, each molecule of which has attached thereto a polymerizable unsaturated group.

However, the polarizers made from set suspensions of the particles and other materials of the present invention will be stable to high levels of heat and ultraviolet radiation and will tolerate water excellently. Accordingly, the present invention makes possible a substantial improvement in the quality of light-polarizing bodies and products incorporating such materials.

Although specific embodiments of the invention have been described, it will be appreciated that many modifications thereon may be made by one skilled in the art, which fall within the spirit and scope of this invention.

## Claims

1. A light-polarizing material containing adsorbed iodine, characterized by said material comprising a complex obtained by reacting (i) elemental iodine, (ii) a hydrohalide acid and/or an ammonium or alkali metal or alkaline earth metal halide and (iii) a compound having the formula:

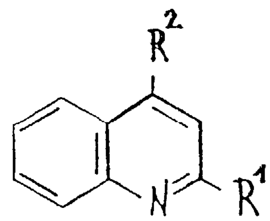

wherein $R^1$ is carboxy and $R^2$ is methoxy, hydroxy or lower alkyl, having 1 to 6, preferably 1 to 4 carbon atoms substituted by hydroxy or methoxy.

2. The light-polarizing material according to claim 1, characterized in that said halide of said hydrohalide acid and/or said ammonium or alkali metal or alkaline earth metal halide is chloride, bromide or iodide.

3. The light-polarizing material according to claim 1 or 2, characterized in that said compound is 4-methoxy quinaldic acid or 4-hydroxy quinaldic acid.

4. A liquid suspension for a light valve, comprising an electrically resistive liquid suspending medium, a plurality of small, anisometrically shaped particles of a light-polarizing material dispersed therein and at least one dispersing material dissolved therein for dispersing said particles in said suspension, characterized in that said light-polarizing particles are particles of the light-polarizing material according to claim 1, 2 or 3.

5. A light-polarizing body, comprising a plurality of particles of a light-polarizing material dispersed in a carrier, the polarizing axis of said particles being oriented and immovably retained by said carrier in substantial parallelism, characterized in that said light-polarizing particles are particles of the light-polarizing material according to claim 1, 2 or 3.

6. A light valve, comprising a cell containing a suspension of light-polarizing particles in a liquid suspending medium, characterized in that said light-polarizing particles are particles of the light-polarizing material according to claim 1, 2 or 3.

## Patentansprüche

1. Ein adsorbiertes Jod enthaltendes, das Licht polarisierendes Material, welches dadurch gekennzeichnet ist, daß das besagte Material einen Komplex umfasst, den man durch eine Umsetzung der

folgenden Verbindungen miteinander erhält: (i) elementares Jod, (ii) eine Halogenwasserstoffsäure und/oder ein Ammoniumhalogenid oder ein Alkalimetallhalogenid oder ein Erdalkalimetallhalogenid und (iii) eine Verbindung, welche die nachfolgend wiedergegebene, allgemeine Formel aufweist:

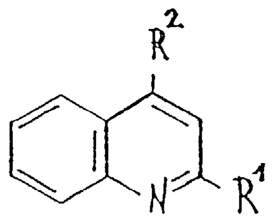

in welcher vorstehenden, allgemeinen Formel die funktionelle Gruppe mit der allgemeinen Bezeichnung $R^1$ einer Carboxylgruppe entspricht, während die funktionelle Gruppe mit der allgemeinen Bezeichnung $R^2$ einer Methoxygruppe oder einer Hydroxylgruppe oder einer kurzkettigen Alkylgruppe entspricht, welch letztere 1 bis 6, vorzugsweise 1 bis 4 Kohlenstoffatome aufweist und außerdem durch eine Hydroxylgruppe oder eine Methoxygruppe substituiert ist.

**2.** Ein das Licht polarisierendes Material entsprechend dem Anspruch 1, dadurch gekennzeichnet, daß es sich bei dem erwähnten Halogenid der besagten Halogenwasserstoffsäure und/oder des Ammoniumhalogenids oder des Alkalimetallhalogenids oder des Erdalkalimetallhalogenids jeweils entweder um das Chlorid, das Bromid oder das Jodid handelt.

**3.** Ein das Licht polarisierendes Material entsprechend einem der Ansprüche 1 oder 2, dadurch gekennzeichnet, daß es sich bei der besagten Verbindung entweder um die Verbindung 4-Methoxychinaldinsäure oder um die Verbindung 4-Hydroxychinaldinsäure handelt.

**4.** Eine flüssige Suspension, die für ein Lichtventil geeignet ist und welche folgendes umfasst: ein flüssiges, suspendierendes Medium, das einen elektrischen Widerstand aufweist, eine Vielzahl kleiner, anisometrisch geformter Teilchen, die aus einem das Licht polarisierenden Material bestehen und welche Teilchen in dem besagten, suspendierenden Medium verteilt sind, außerdem mindestens ein in dem besagten, suspendierenden Medium aufgelöstes, dispergierendes Material zum Verteilen der erwähnten Teilchen in der besagten Suspension, wobei diese besagte Suspension dadurch gekennzeichnet ist, daß es sich bei den erwähnten, das Licht polarisierenden Teilchen um Teilchen handelt, die aus dem das Licht polarisierenden Material entsprechend einem der Ansprüche 1, 2 oder 3 bestehen.

**5.** Ein das Licht polarisierender Körper, der eine Vielzahl von Teilchen umfaßt, die aus einem das Licht polarisierenden Material bestehen und die in einem Trägermaterial verteilt sind, wobei die polarisierenden Achsen der besagten Teilchen jeweils orientiert sind und durch das besagte Trägermaterial unbeweglich in einer im wesentlichen zueinander parallel ausgerichteten Lage festgehalten werden und wobei der besagte Körper dadurch gekennzeichnet ist, daß es sich bei den erwähnten, das Licht polarisierenden Teilchen um Teilchen handelt, die aus dem das Licht polarisierenden Material entsprechend einem der Ansprüche 1, 2 oder 3 bestehen.

**6.** Ein Lichtventil, welches eine Zelle umfasst, die eine Suspension auf der Basis von das Licht polarisierenden Teilchen enthält, die in einem flüssigen, suspendierenden Medium verteilt sind, und welches Lichtventil dadurch gekennzeichnet ist, daß es sich bei den erwähnten, das Licht polarisierenden Teilchen um Teilchen handelt, die aus dem das Licht polarisierenden Material entsprechend einem der Ansprüche 1, 2 oder 3 bestehen.

**Revendications**

**1.** Matériau polarisant la lumière contenant de l'iode adsorbé, caractérisé en ce que ledit matériau comprend un complexe obtenu par réaction (i) d'iode élémentaire, (ii) d'un acide halogénhydrique et/ou d'un halogénure d'ammonium ou alcalin ou alcalino-terreux et (iii) d'un composé de formule :

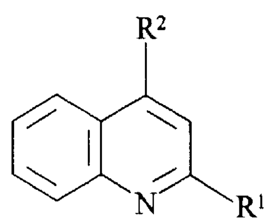

où $R^1$ est carboxyle et $R^2$ est méthoxy, hydroxyle ou alkyle inférieur, ayant 1 à 6, de préférence 1 à 4 atomes de carbone, substitué par hydroxyle ou méthoxy.

**2.** Matériau polarisant la lumière selon la revendication 1, caractérisé en ce que ledit halogénure dudit acide halogénhydrique et/ou ledit halogénure d'ammonium ou alcalin ou alcalino-terreux est un chlorure, un bromure ou un iodure.

**3.** Matériau polarisant la lumière selon la revendication 1 ou 2, caractérisé en ce que ledit composé est l'acide 4-méthoxyquinaldique ou l'acide 4-hydroxyquinaldique.

**4.** Suspension liquide pour modulateur de lumière comprenant un milieu de suspension liquide électriquement résistant, une pluralité de petites particules de forme anisométrique d'un matériau polarisant la lumière dispersées dans celui-ci et au moins un matériau dispersant dissous dans celui-ci pour disperser lesdites particules dans ladite suspension, caractérisée en ce que lesdites particules polarisant la lumière sont des particules du matériau polarisant la lumière selon la revendication 1, 2 ou 3.

**5.** Corps polarisant la lumière comprenant une pluralité de particules d'un matériau polarisant la lumière dispersées dans un support, l'axe polarisant lesdites particules étant orienté et maintenu de manière immobile par ledit support en parallélisme sensible, caractérisé en ce que lesdites particules polarisant la lumière sont des particules du matériau polarisant la lumière selon la revendication 1, 2 ou 3.

**6.** Modulateur de lumière comprenant une cellule contenant une suspension de particules polarisant la lumière dans un milieu de suspension liquide, caractérisé en ce que lesdites particules polarisant la lumière sont des particules du matériau polarisant la lumière selon la revendication 1, 2 ou 3.